# EUROPEAN PATENT SPECIFICATION

(11) **EP 4 253 563 B1**
(45) Date of publication and mention of the grant of the patent: **28.01.2026**
(21) Application number: 22164966.8
(22) Date of filing: 29.03.2022
(51) Int. Cl.: C12Q 1/6853

(54) **PHOTO-RESPONSIVE OLIGONUCLEOTIDES**
LICHTEMPFINDLICHE OLIGONUKLEOTIDE
OLIGONUCLÉOTIDES PHOTOSENSIBLES

(43) Date of publication of application: 04.10.2023
(73) Proprietor: Miltenyi Biotec B.V. & Co. KG, 51429 Bergisch Gladbach (DE)
(72) Inventor: PERBOST, Michel, 51429 Bergisch Gladbach (DE); PINARD, Robert, 51429 Bergisch Gladbach (DE); HOSONO, Seiyu, 51429 Bergisch Gladbach (DE); NEIL, Emily, 51429 Bergisch Gladbach (DE)

(56) References cited:
- EP-A1- 2 878 671
- EP-B1- 3 015 555
- WO-A1-01/21637
- JP-A- 2021 175 393
- JP-A- 2021 175 736
- US-A1- 2015 093 836
- YAMANA K ET AL: "Synthesis of A New Photoisomerizable Linker for Connecting Two Oligonucleotide Segments", TETRAHEDRON LETTERS, ELSEVIER, AMSTERDAM , NL, vol. 37, no. 5, 29 January 1996 (1996-01-29), pages 637 - 640, XP004030320, ISSN: 0040-4039, DOI: 10.1016/0040-4039(95)02220-1
- NIKOLAI GREBENOVSKY ET AL: "Introducing LNAzo: More Rigidity for Improved Photocontrol of Oligonucleotide Hybridization", CHEMISTRY - A EUROPEAN JOURNAL, JOHN WILEY & SONS, INC, DE, vol. 25, no. 53, 28 August 2019 (2019-08-28), pages 12298 - 12302, XP071849331, ISSN: 0947-6539, DOI: 10.1002/CHEM.201903240
- GREBENOVSKY NIKOLAI ET AL: "Introducing LNAzo: More Rigidity for Improved Photocontrol of Oligonucleotide Hybridization: Supportive Information", CHEMISTRY - A EUROPEAN JOURNAL, vol. 25, no. 53, 20 September 2019 (2019-09-20), DE, pages 12298 - 12302, XP093144166, ISSN: 0947-6539, DOI: 10.1002/chem.201903240
- YOUNG DOUGLAS D. ET AL: "Light-triggered polymerase chain reaction", CHEMICAL COMMUNICATIONS, no. 4, 1 January 2008 (2008-01-01), UK, pages 462 - 464, XP093034934, ISSN: 1359-7345, Retrieved from the Internet <URL:https://www.ncbi.nlm.nih.gov/pmc/articles/PMC3760149/pdf/nihms494938.pdf> DOI: 10.1039/B715152G

## Description

### Field of invention

The present invention relates to the field of nucleic acid analysis, spatial transcriptomics and next generation sequencing.

### Background

The analysis of nucleic acids is an important tool in molecular biology. Typical applications are next generation sequencing and spatial transcriptomics. All methods rely on specific binding of oligonucleotides that are either used for detection of specific sequences in a sample or as starting point of a nucleic acid synthesis reaction for amplification or sequencing. Prominent examples of these techniques are exemplary disclosed in: smFISH (A. M. Femino, F. S. Fay, K. Fogarty, R. H. Singer, Science 1998, 280, 585; A. Raj, P. Van Den Bogaard, S. Rifkin, Nat. Methods 2008, 5, 877), MERFISH (K. H. Chen, A. N. Boettiger, J. R. Moffitt, S. Wang, X. Zhuang, Science 2015, 348, aaa6090; G. Wang, J. R. Moffitt, X. Zhuang, Sci. Rep. 2018, 8, 4847; F. Chen, A. T. Wassie, A. J. Cote, A. Sinha, S. Alon, S. Asano, E. R. Daugharthy, J. B. Chang, A. Marblestone, G. M. Church, A. Raj, E. S. Boyden, Nat. Methods 2016, 13, 679), smHCR (S. Shah, E. Lubeck, M. Schwarzkopf, T. F. He, A. Greenbaum, C. H. Sohn, A. Lignell, H. M. Choi, V. Gradinaru, N. A. Pierce, L. Cai, Development 2016, 143, 2862), seqFISH (E. Lubeck, A. F. Coskun, T. Zhiyentayev, M. Ahmad, L. Cai, Nat. Methods 2014, 11, 360; S. Shah, E. Lubeck, W. Zhou, L. Cai, Neuron 2016, 92, 342.), seqFISH+ (C.-H. L. Eng, M. Lawson, Q. Zhu, R. Dries, N. Koulena, Y. Takei, J. Yun, C. Cronin, C. Karp, G. C. Yuan, L. Cai, Nature 2019, 568, 235), osmFISH (S. Codeluppi, L. E. Borm, A. Zeisel, G. La Manno, J. A. van Lunteren, C. I. Svensson, S. Linnarsson, Nat. Methods 2018, 15, 932), RNAscope (D. Clair, Bio-Techne Announces Commercial Release of RNAscope® HiPlex Assay: A Multiplex In Situ Hybridization Assay For Tissues 2016; D. Schulz, V. R. T. Zanotelli, J. R. Fischer, D. Schapiro, S. Engler, X. K. Lun, H. W. Jackson, B. Bodenmiller, Cell Syst. 2018, 6, 25. e5; A. Mavropoulos, B. Allo, M. He, E. Park, D. Majonis, O. Ornatsky, Cytometry, Part A 2017, 91, 1200.)

Spatial transcriptomics applications focus on nucleic acid analysis in e.g. a tissue sample. Primer (oligonucleotides) are used to start a nucleic acid synthesis reaction and sequencing reaction. Added molecules bind to all complementary nucleic acids in all locations in the sample, without the ability to control hybridization to an area of interest. In addition to that using the sample for multiple rounds of analysis is difficult, because existing primers need to be removed under harsh conditions. This often leads to sample damage and remaining contaminates, which interfere with downstream reactions.

Oligonucleotide hybridization and removal is typically a function of the melting temperature of a dimer (Tm). This temperature is a function of length and sequence of the nucleic acid; %GC; type of nucleic acids (RNA or DNA) and modifications (a small number among a myriad: phosphorothioate, 2'Fluoro, LNA; Nucleic Acids Research, 2007, Vol. 35, Web Server issue W43-W46; Howley PM, Israel MF, Law M-F, Martin MA. A rapid method for detecting and mapping homology between heterologous DNAs. Evaluation of polyomavirus genomes. J. Biol. Chem. 1979;254:4876-4883; Breslauer KJ, Frank R, Blöcker H, Marky LA. Predicting DNA duplex stability from the base sequence. Proc. Natl Acad. Sci. USA. 1986;83:3746-3750; Sugimoto N, Nakano S, Yoneyama M, Honda K. Improved thermodynamic parameters and helix initiation factor to predict stability of DNA duplexes. Nucleic Acids Res. 1996;24:4501-4505). Based on that oligonucleotide hybridization can be controlled by salt concentration (monovalent like Na+ or bivalent like Mg2+), temperature, presence or absence of crowding agent PEG, dextran); presence or absence of destabilizing agent (formamide; ethylene carbonate, urea) and pH specific buffer conditions (Lee, Je Hyuk, et al. "Fluorescent in situ sequencing (FISSEQ) of RNA for gene expression profiling in intact cells and tissues." Nature protocols 10.3 (2015): 442-458.; MERFISH (K. H. Chen, A. N. Boettiger, J. R. Moffitt, S. Wang, X. Zhuang, Science 2015, 348, aaa6090; G. Wang, J. R. Moffitt, X. Zhuang, Sci. Rep. 2018, 8, 4847; seqFISH+ (C.-H. L. Eng, M. Lawson, Q. Zhu, R. Dries, N. Koulena, Y. Takei, J. Yun, C. Cronin, C. Karp, G. C. Yuan, L. Cai, Nature 2019, 568, 235), osmFISH (S. Codeluppi, L. E. Borm, A. Zeisel, G. La Manno, J. A. van Lunteren, C. I. Svensson, S. Linnarsson, Nat. Methods 2018, 15, 932).

One of the major drawbacks of the existing methods is the introduction of contaminants into the reaction system by specific buffer components, which may lead to sample damage or interfere with potential downstream reactions. Moreover, currently there is low controllability of localized oligonucleotide binding or localized oligonucleotide removal.

The inventors found that this problem can be solved by the use of photo-responsive oligonucleotides. The use of light (ultraviolet or visible) to control a reaction, has a number of advantages over other external stimuli: (1) Light does not introduce contaminants into the reaction system; (2) Excitation wavelength can be controlled through the design of the photo-responsive molecule; (3) control irradiation time and/or local excitation.

Applications of DNA-mediated bioprocesses using photo responsive DNA elements have been disclosed by H. Asanuma,et al. and others (Angew. Chem. Int. Ed. 2001, 40, 2671-2673, H. Asanuma, et al., Nat. Protoc. 2007, 2, 203-212; Y.Kamiya and H. Asanuma, Acc. Chem. Res. 2014, 47, 1663-1672; X.G. Liang, et al., J. Am. Chem. Soc. 2003, 125, 16408-16415; H. Ito, et al., Org. Biomol. Chem. 2010, 8, 5519-5524; X.G. Liang, et al., J. Am. Chem. Soc. 2002, 124, 1877-1883; M. Zhou, et al., Angew. Chem., Int. Ed. 2010, 49, 2167-2170; M. Liu, et al., J. Am. Chem. Soc. 2003, 128, 1009-1015). They describe the modification of nucleic acids by azobenzene derivatives and their applications in biotechnology and nanotechnology. In addition to that, EP3015555B1 describes the use of photo-responsive nucleic acid templates for isothermal DNA amplification. Young et al. (28-01-2008) teaches hairpin priming where photolithic caging groups are placed at specific locations on the primer but are eliminated under the action of UV. However, documents fail to describe the use of photo-responsive oligonucleotides for nucleic acid analysis, spatial transcriptomics and next generation sequencing.

The inventors surprisingly found that photo-responsive oligonucleotides allow a controlled binding and gentle removal of these structures without using harsh conditions, while preserving sample integrity. Moreover they found that photo-responsive oligonucleotides provide great potential to spatially control their binding in a sample.

### Summary of the invention

The object of the present invention is to provide a method for hybridization of a photo-responsive oligonucleotide to a nucleic acid by providing the nucleic acid with a complementary oligonucleotide, wherein the oligonucleotide functions as a starting point for a polymerase for nucleic acid synthesis **characterized in that** the photo-responsive oligonucleotide comprises at least two photo-responsive elements which change from a first to a second conformation upon irradiation with light thereby disabling or enabling the oligonucleotide hybridization.

In addition to that, the current invention provides a method for spatially controlled oligonucleotide hybridization to specific sites by spatial illumination of areas of no interest, thus changing the oligonucleotide conformation to a non-binding state.

The reversable hybridization of the oligonucleotide can be used for controlling several reactions such as rolling circle amplification and a sequencing reaction.

### Legends of the drawings

The drawings shall explain the method of the invention and its embodiments without limiting the scope of the claims.

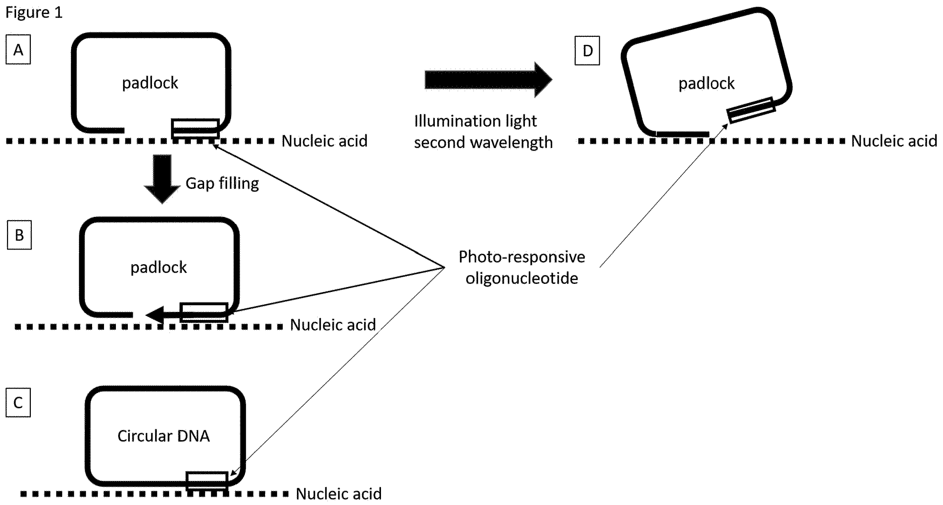

Figure 1 shows an example of the photo-responsive oligonucleotide which is part of a padlock system. The nucleic acid e.g. a mRNA serves as template for a nucleic acid synthesis (gap filling) reaction (figure 1A). After binding of the padlock to the nucleic acid, the padlock may be removed from the nucleic acid by illumination with light having a second wavelength as disclosed herein. This induces a conformational change of the oligonucleotide to a non-binding state, which leads to the unbinding of said padlock (figure 1D). Then the gap of the padlock, not illuminated with the second wavelength, and therefore still bound to the nucleic acid, is filled with complementary nucleotides and ligated (figure 1B). By doing so a circular template is formed (figure 1C), which comprises said photo-responsive oligonucleotide.

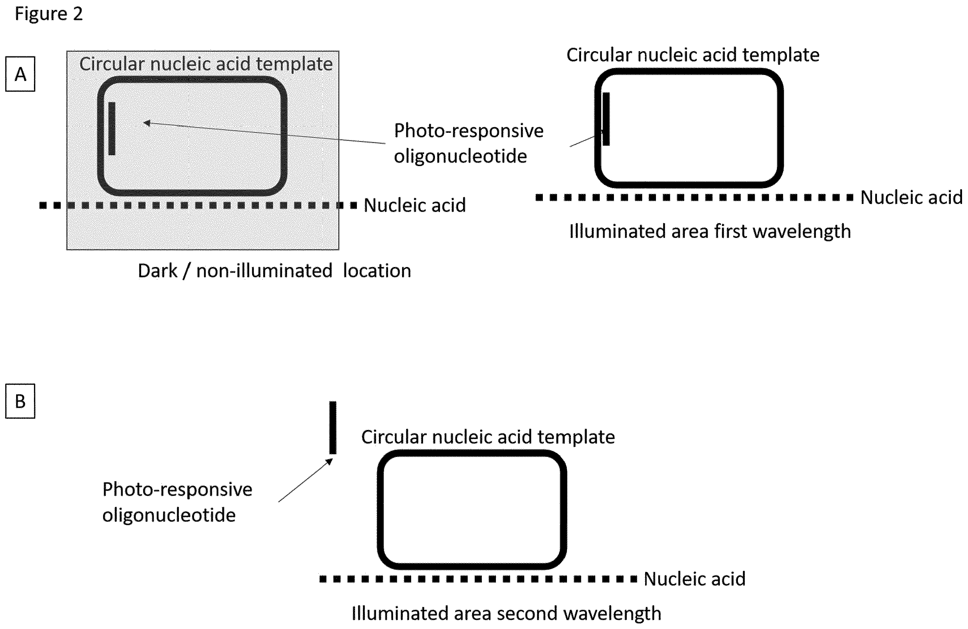

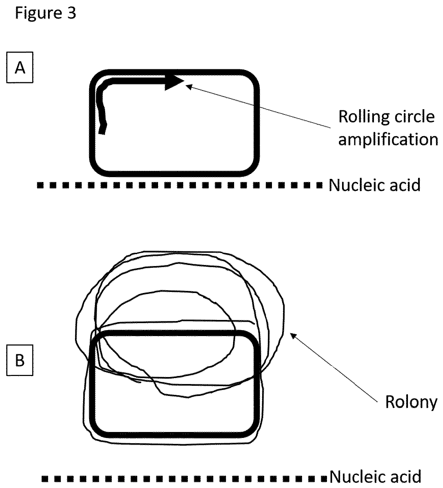

Figure 2 and figure 3 illustrate a spatially controlled rolling circle nucleic acid synthesis reaction. Photo-responsive oligonucleotides are added to a sample containing circular nucleic acid templates e.g. provided by the padlock system. Areas of no interest in the sample are illuminated with light having a second wavelength as disclosed herein. As a response, oligonucleotides localized in these regions change conformation to a non-binding state. Therefore said oligonucleotides do not bind or dissociate if already bound from the nucleic acid, thus preventing a nucleic acid synthesis reaction (figure 2B). Photo-responsive oligonucleotides localized in non-illuminated areas or in areas of the sample that have been illuminated with light having a first wavelength as disclosed herein, keep the first conformational state (binding state; figure 2A). In regions where the photo-responsive oligonucleotide is bound to the template a rolling circle amplification is initiated. Based on rolling circle amplification a nucleic acid (rolony) is generated comprising several copies of the initial circular nucleic acid template (figure 3).

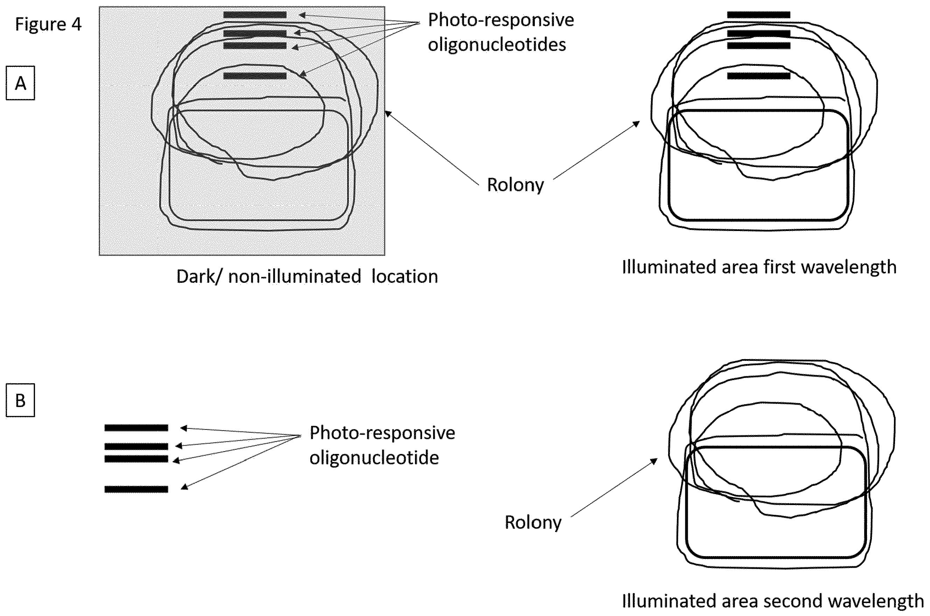

Figure 4 shows spatially controlled oligonucleotide binding for nucleic acid synthesis using rolonies as nucleic acid template. Photo-responsive oligonucleotides are added to a sample containing nucleic acid templates provided by the rolonies. There follows illumination of areas of no interest in the sample with light having a second wavelength as disclosed herein. Oligonucleotides localized in these regions change conformation to a non-binding state. This leads to dissociation or non-binding of said oligonucleotides from said nucleic acid thus preventing a nucleic acid synthesis reaction (figure 4B). Photo-responsive oligonucleotides localized in non-illuminated areas or in areas of the sample that have been illuminated with light having the first wavelength as disclosed herein, keep the first conformational state (binding state; figure 4A). In these regions the photo-responsive oligonucleotide binds to the template and a nucleic acid synthesis reaction is initiated.

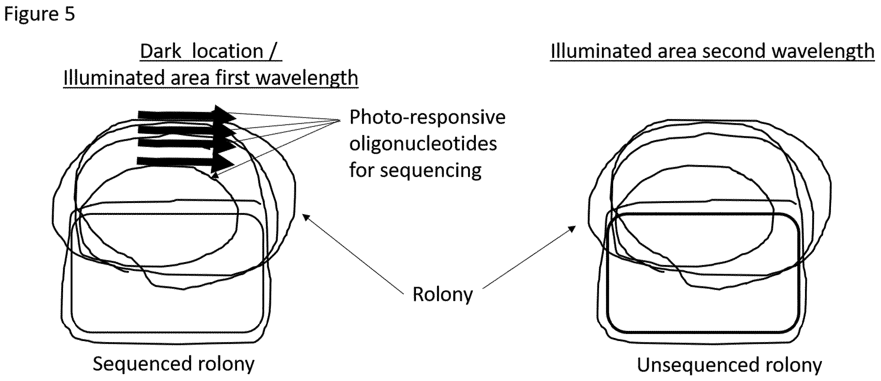

Figure 5 exemplary shows a spatially controlled sequencing reaction in which a rolony serves as template. Oligonucleotide binding is enabled in non-illuminated areas of the sample or in areas that been illuminated with light having a first wavelength, as disclosed herein. This then leads to the initiation of said sequencing reaction. No reaction is initiated in areas that have been illuminated with light having a second wavelength, because no oligonucleotide binding is possible in these areas.

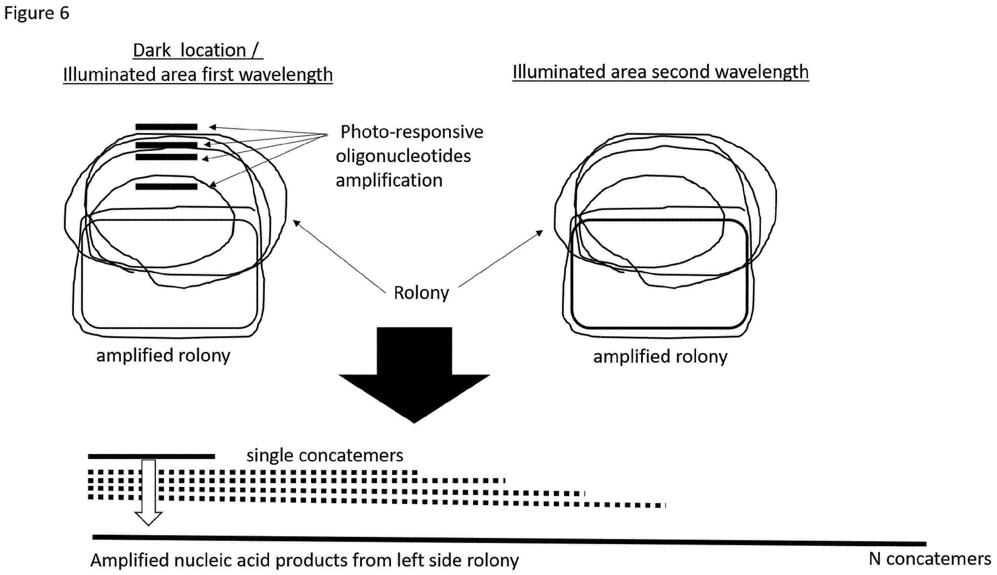

Figure 6 exemplary shows a spatially controlled nucleic acid amplification reaction. Oligonucleotide binding is enabled in non-illuminated areas of the sample or in areas that been illuminated with light having a first wavelength. as disclosed herein. This then leads to the initiation of said nucleic acid amplification reaction. No reaction is initiated in areas that have been illuminated with light having a second wavelength, because no oligonucleotide binding is possible in these areas. As a result of such an amplification reaction, amplified nucleic acid products (concatamers) are generated.

### Detailed description

The present invention provides a method for reversable and spatially controlled hybridization of photo-responsive oligonucleotides to nucleic acids in a sample by using light. Photo-responsive oligonucleotides can thus be removed under gentle conditions, which leads to increased preservation of sample integrity, thus enabling multiple rounds of analysis.

In a first aspect, the invention provides a method for hybridization of an oligonucleotide to a nucleic acid by providing the nucleic acid with a complementary oligonucleotide. Said oligonucleotide functions as a starting point for a polymerase for nucleic acid synthesis characterized in that the oligonucleotide comprises at least two photo-responsive elements. These photo-responsive elements can change from a first to a second conformation upon irradiation with light, thereby disabling or enabling the oligonucleotide hybridization.

### Photo-responsive oligonucleotides

Several photo-responsive structures are known in the art as molecular photoswitches. They are defined as chemical structures that convert to two or more isomers under light irradiation. The isomerization between chemical structure proceeds via two basic mechanisms: trans to cis isomerization and 6 Π electrocyclization of a triene system. Several photo-responsive structures are known in the art such as azobenzenes and their heteroaromatic analogues, indigos, hemithioindigos, stilbenes, hydrazones, iminothioindoxyls, diarylethenes, Spiropyrans, Stenhouse adducts (DASAs)15 and fulgides ( Ilse M. Welleman, Mark W. H. Hoorens, Ben L. Feringa, Hendrikus H. Boersma and Wiktor Szymanski Chem. Sci., 2020, 11, 11672-11691).

Azobenzene derivatives are the most popular photo-responsive molecules for versatile applications because of their ready availability and chemical stability. A planar trans form can be obtained upon photo-irradiation at one wavelength and a nonplanar cis form is obtained by photo-irradiation at another wavelength. Therefore, azobenzene can reversibly photo-isomerize between a first conformation (trans) and a second conformation (cis) upon irradiation with the appropriate wavelength of light.

A phosphoramidite monomer bearing an azobenzene group covalently and which is connected through an amide bond to D-threoninol as the scaffold can be synthetized as described in: Enantioselective Incorporation of Azobenzenes into Oligodeoxyribonucleotide for Effective Photoregulation of Duplex Formatio ( H. Asanuma, T. Takarada, T. Yoshida, D. Tamaru, X. Liang et M. Komiyama, Angewandte Chemie-International Edition 2001, 40, 2671-2673).

Use of this monomer allows the simple introduction of an azobenzene cartridge as photo-responsive element (X) into the oligonucleotide, thus making it photo-responsive. Therefore according to the current invention the terms "oligonucleotide" and "photo-responsive oligonucleotide" may be used interchangeably.

Based on the properties of the azobenzene cartridge, photo-regulation of the oligonucleotide is achieved. The photo-responsive oligonucleotide changes from a first to a second conformation upon irradiation with light having a first wavelength and from the second to the first conformation upon irradiation with light having a second wavelength. According to the current invention the first (initial) conformation of the photo-responsive oligonucleotide is trans. The terms may be used interchangeably. In this form the oligonucleotide can hybridize to a nucleic acid. Contrary to that, an oligonucleotide in the second conformational state (cis form) cannot bind, due to steric hinderance. The second conformational state is also termed "non-binding state" or "cis". According to the current invention the terms "hybridization", "binding" and their grammatical equals may be used interchangeably.

A conformational change of the oligonucleotide as disclosed herein, to the second conformation can be induced by illumination with light of a first wavelength. The first wavelength is in a range of 300-400nm, more preferentially 330nm. It has to be noted that the conformational change is reversable, i.e. the conformation can be changed from the second conformation (cis) to the first conformation (trans) by illumination with light of a second wavelength. The second wavelength is in a range of 400-600nm, more preferentially 532 or 525 nm.

To provide the functionality of azobenzene in the oligonucleotide, the photo-responsive element (X) is introduced between base pairs (N) of the nucleic acid ( e.g. NNN → NXNN). The modified photo-responsive oligonucleotide strand can still form a duplex with its complementary strand, and all base pairs are maintained in the duplex. In order to attain efficient photo-regulation, introduction of multiple azobenzene residues is effective. It should be noted that replacing a natural nucleotide with X is not recommended as it causes destabilization of the duplex.

In one embodiment of the invention the structure of the photo-responsive oligonucleotide has a ratio of X:N of 1:2 or 1:3 , wherein N is a nucleotide, X is a photo-responsive element. In a more preferred embodiment of the invention the photo-responsive oligonucleotide has the formula N(XNN)nXN, wherein N is a nucleotide, X is a photo-responsive element and n = 3-29, preferentially 9, 14 or 23. Using this design strategy, repetitive and efficient regulation of hybridization and dissociation of the nucleotide duplex can be promoted by alternating illumination between first and second wavelength. The photo-responsive oligonucleotide has a length of at least 15, 50 or 70, more preferentially 20 or 30 or 50 nucleotides.

### Method / Applications

The photo-responsive oligonucleotide can be hybridized to complementary parts of a nucleic acid. It may serve as starting point for a polymerase for nucleic acid synthesis.

Said nucleic acid may serve as template for the generation of a complementary nucleotide strand by a polymerase, thereby creating a synthetized nucleic acid strand (nucleic acid synthesis). During nucleic acid synthesis, complementary nucleotides are add to the 3' end of the bound oligonucleotide by a polymerase, thus, generating a complementary nucleic acid. Standard polymerases that can be used for these reactions are e.g. T7 DNA polymerase, Klenow Fragment, T9 DNA polymerase.

Nucleic acid synthesis reactions, wherein said nucleic acid served as template, include but are not limited to cDNA synthesis, linear DNA amplification, sequencing, gap filling reactions or rolling circle amplification.

The template for the reaction is provided by the nucleic acid in the sample e.g. tissue sample, a chip or a hydrogel. The nucleic acid may be a polynucleotide strand made of deoxyribonucleic acid (DNA) or ribonucleic acid (RNA), which may have a linear or circular conformation. In a specific embodiment of the invention the nucleic acid is circular and serves as template for rolling circle amplification.

Samples to be analyzed with the disclosed methods may originate from any specimen, like whole animals, organs, tissues slices, cell aggregates, or single cells of invertebrates, (e.g., Caenorhabditis elegans, Drosophila melanogaster), vertebrates (e.g., Danio rerio, Xenopus laevis) and mammalians (e.g., Mus musculus, Homo sapiens). A biological sample may have the form of a tissues slice, cell aggregate, suspension cells, or adherent cells.

In one embodiment of the invention, the photo-responsive oligonucleotide may be used for non-destructive removal of synthetized nucleic acid strands. After hybridization of the photo-responsive oligonucleotide to the nucleic acid, a nucleic acid synthesis reaction is initiated, thus generating a synthetized nucleic acid strand comprising said photo-responsive oligonucleotide. The conformation of the photo-responsive oligonucleotide is changed to a non-binding state upon irradiation with light, thus allowing the synthetized nucleic acid to dissociate from the template.

In another embodiment of the invention the photo-responsive oligonucleotide is part of a padlock system as disclosed in EP3936623A1 (figure 1). The nucleic acid serves as template for the gap filling reaction (figure 1A). After binding of the padlock to the nucleic acid the gap of the padlock is filled with complementary nucleotides and ligated (figure 1B). By doing so a circular template is formed (figure 1C). It comprises said photo-responsive oligonucleotide. This template may be further used for nucleic acid synthesis reactions.

In one embodiment the padlock may be removed from the nucleic acid by illumination with light having a second wavelength as disclosed herein, thus inducing a conformational change of the oligonucleotide to a non-binding state. This may then lead to the unbinding, or partial unbinding, of said padlock (figure 1D).

The invented method may also be used for spacial controlled oligonucleotide hybridization. Oligonucleotide binding may be controlled by spatial illumination of areas of no interest. This leads to a conformational change of the oligonucleotide to a non-binding state, thereby controlling oligonucleotide hybridization to specific sites. In more detail, during illumination with a first wavelength a conformational change of the photo-responsive oligonucleotides to the second conformational state (cis) is initiated in the illuminated regions. This then leads to selective removal of the oligonucleotides.

In another embodiment areas of no interest may be irradiated with light of a first wavelength before and / or during oligonucleotide binding, thus generating a light based mask. Because in these areas oligonucleotides would have a second conformation (non-binding) only, their binding to the illuminated areas will be prevented.

In one embodiment spatially controlled oligonucleotide hybridization may be used for spatially controlled nucleic acid synthesis. Photo-responsive oligonucleotides are provided to a sample comprising nucleic acids. There follows controlled spatial illumination of areas of no interest with light having a second wavelength, as disclosed herein. This induces a conformational change to a non-binding state of said oligonucleotides in areas of no interest. These oligonucleotides cannot bind or dissociate if already bound from the nucleic acids in these areas. Contrary to that oligonucleotides in non-illuminated regions or regions that were illuminated with light having the first wavelength, as disclosed herein, are in a binding state. Therefore binding to the nucleic acids is enabled. After binding a nucleic acid synthesis can be initiated in these areas.

**In** a specific embodiment of the invention said spatially controlled nucleic acid synthesis reaction may be a rolling circle amplification as exemplary represented in figure 2 and figure 3. Herein photo-responsive oligonucleotides are provided to circular nucleic acid templates in a sample e.g. provided by the padlock system. Areas in the sample which are of no interest are illuminated with light having a second wavelength, as disclosed herein. Oligonucleotides localized in these regions change conformation to a non-binding state. This leads to unbinding (dissociation) of these oligonucleotides thus preventing a nucleic acid synthesis reaction (figure 2B). Contrary to that conditions for photo-responsive oligonucleotide binding are provided in non-illuminated areas or in areas of the sample that have been illuminated with the first wavelength as disclosed herein, thus keeping the oligonucleotide in the first conformational state, the binding state (figure 2A). In regions where the photo-responsive oligonucleotide is bound to the template a rolling circle amplification may be initiated. Based on rolling circle amplification a nucleic acid is generated comprising several copies of the initial circular nucleic acid template (figure 3B). This structure is defined as rolony. In another embodiment of the invention said rolony may serve as template for a spatially controlled nucleic acid synthesis reaction such as nucleic acid amplification or sequencing (figure 4).

**In** a specific embodiment, the invention may be used for a spatially controlled sequencing reaction in which a rolony serves as a template. Oligonucleotide binding is enabled in non-illuminated areas of the sample or in areas that been illuminated with light having a first wavelength, as disclosed herein. A sequencing reaction can be initiated. No reaction can be initiated in areas that have been illuminated with light having a second wavelength, because oligonucleotide binding is disabled in these areas (figure 5).

In another specific embodiment, the invention may be used for a spatially controlled nucleic acid amplification reaction. Oligonucleotide binding is enabled in non-illuminated areas of the sample or in areas that been illuminated with light having a first wavelength as disclosed herein. This then leads to the initiation of said nucleic acid amplification reaction. No reaction is initiated in areas that have been illuminated with light having a second wavelength, because no oligonucleotide binding is possible in these areas. As a result of such an amplification reaction, amplified nucleic acid products (concatamers) are generated (figure 6).

## Claims

1. A method for hybridization of at least one oligonucleotide to a nucleic acid by providing the nucleic acid with at least one complementary oligonucleotide, wherein the oligonucleotide functions as a starting point for a polymerase for nucleic acid synthesis **characterized in that** the oligonucleotide comprises at least two photo-responsive elements which change from a first to a second conformation upon irradiation with light, thereby disabling or enabling the oligonucleotide hybridization.

2. A method according to claim 1 **characterized in that** the nucleic acid serves as template for the generation of a complementary nucleotide strand by a polymerase thereby creating a synthetized nucleic acid strand.

3. A method according to claim 1 or 2 **characterized in that** the nucleic acid is circular and serves as template for rolling circle amplification.

4. A method according any of the claims 1 - 3 **characterized in that,** the photo-responsive oligonucleotide is part of a padlock system and the nucleic acid serves as template for the gap filling reaction, wherein the gap of the padlock is filled with complementary nucleotides and ligated, a circular template is formed.

5. A method according to claim 2 or 4 **characterized in that** the synthetized nucleic acid strand comprises the photo-responsive oligonucleotide, wherein the conformation of the photo-responsive oligonucleotide is changed to a non-binding state upon irradiation with light, thus allowing the synthetized nucleic acid to dissociate from the template.

6. A method according to claim 1 **characterized in that** the provided nucleic acid serves as template for a sequencing reaction.

7. A method according to any of the claims 1-6 **characterized in that** oligonucleotide binding is controlled by spatial illumination of areas of no interest, thus changing the oligonucleotide conformation to a non-binding state, thereby controlling oligonucleotide hybridization to specific sites

8. A method according to any of the claims 1-7 **characterized in that** the length of the photo-responsive oligonucleotide is at least 15, 50 or 75 nucleotides

9. A method according to any of the claims 1-8 **characterized in that** the structure of the photo-responsive oligonucleotide has a ratio of X:N of 1:2 or 1:3 , wherein N is a nucleotide, X is a photo-responsive element

10. A method according to any of the claims 1-9 **characterized in that** photo-responsive oligonucleotide has the formula N(XNN)nXN, wherein N is a nucleotide, X is a photo-responsive element and n = 3-29

11. A method according to any of the claims 1-10 **characterized in that** photo-responsive oligonucleotide changes from a first to a second conformation upon irradiation with light having a first wavelength and from the second to the first conformation upon irradiation with light having a second wavelength

12. A method according to claim 11 **characterized in that** the first wavelength is between 300-400 nm and the second wavelength is 400-600 nm

13. A method according to any of the claims 1-12 **characterized in that** the nucleic acid is provided by a tissue sample, a chip or a hydrogel

14. A method according to any of the claims 1-13 **characterized in that** the nucleic acid is linear or circular

## Patentansprüche

1. Verfahren zur Hybridisierung mindestens eines Oligonukleotids mit einer Nukleinsäure, indem die Nukleinsäure mit mindestens einem komplementären Oligonukleotid bereitgestellt wird, wobei das Oligonukleotid als Ausgangspunkt für eine Polymerase zur Nukleinsäuresynthese fungiert, **dadurch gekennzeichnet, dass** das Oligonukleotid mindestens zwei lichtempfindliche Elemente umfasst, die bei Bestrahlung mit Licht von einer ersten in eine zweite Konformation übergehen und dadurch die OligonukleotidHybridisierung deaktivieren oder aktivieren.

2. Verfahren nach Anspruch 1, **dadurch gekennzeichnet, dass** die Nukleinsäure als Matrize für die Erzeugung eines komplementären Nukleotidstrangs durch eine Polymerase dient, wodurch ein synthetisierter Nukleinsäurestrang entsteht.

3. Verfahren nach Anspruch 1 oder 2, **dadurch gekennzeichnet, dass** die Nukleinsäure zirkulär ist und als Matrize für die Rolling-Circle-Amplifikation dient.

4. Verfahren nach einem der Ansprüche 1 bis 3, **dadurch gekennzeichnet, dass** das lichtempfindliche Oligonukleotid Teil eines Padlock-Systems ist und die Nukleinsäure als Matrize für die Lückenfüllungsreaktion dient, wobei die Lücke des Padlocks mit komplementären Nukleotiden gefüllt und ligiert wird, wodurch eine zirkuläre Matrize gebildet wird.

5. Verfahren nach Anspruch 2 oder 4, **dadurch gekennzeichnet, dass** der synthetisierte Nukleinsäurestrang das lichtempfindliche Oligonukleotid umfasst, wobei die Konformation des lichtempfindlichen Oligonukleotids bei Bestrahlung mit Licht in einen nicht bindenden Zustand übergeht, wodurch sich die synthetisierte Nukleinsäure von der Matrize lösen kann.

6. Verfahren nach Anspruch 1, **dadurch gekennzeichnet, dass** die bereitgestellte Nukleinsäure als Matrize für eine Sequenzierungsreaktion dient.

7. Verfahren nach einem der Ansprüche 1 bis 6, **dadurch gekennzeichnet, dass** die Oligonukleotidbindung durch räumliche Beleuchtung von Bereichen ohne Interesse gesteuert wird, wodurch die Oligonukleotidkonformation in einen nicht bindenden Zustand geändert wird, wodurch die Oligonukleotidhybridisierung an spezifische Stellen gesteuert wird.

8. Verfahren nach einem der Ansprüche 1 bis 7, **dadurch gekennzeichnet, dass** die Länge des lichtempfindlichen Oligonukleotids mindestens 15, 50 oder 75 Nukleotide beträgt.

9. Verfahren nach einem der Ansprüche 1 bis 8, **dadurch gekennzeichnet, dass** die Struktur des lichtempfindlichen Oligonukleotids ein Verhältnis von X:N von 1:2 oder 1:3 aufweist, wobei N ein Nukleotid ist und X ein lichtempfindliches Element ist.

10. Verfahren nach einem der Ansprüche 1 bis 9, **dadurch gekennzeichnet, dass** das lichtempfindliche Oligonukleotid die Formel N(XNN)nXN aufweist, wobei N ein Nukleotid ist, X ein lichtempfindliches Element ist und n = 3 bis 29.

11. Verfahren nach einem der Ansprüche 1 bis 10, **dadurch gekennzeichnet, dass** das lichtempfindliche Oligonukleotid bei Bestrahlung mit Licht einer ersten Wellenlänge von einer ersten in eine zweite Konformation und bei Bestrahlung mit Licht einer zweiten Wellenlänge von der zweiten in die erste Konformation übergeht.

12. Verfahren nach Anspruch 11, **dadurch gekennzeichnet, dass** die erste Wellenlänge zwischen 300 und 400 nm liegt und die zweite Wellenlänge zwischen 400 und 600 nm liegt.

13. Verfahren nach einem der Ansprüche 1 bis 12, **dadurch gekennzeichnet, dass** die Nukleinsäure durch eine Gewebeprobe, einen Chip oder ein Hydrogel bereitgestellt wird.

14. Verfahren nach einem der Ansprüche 1 bis 13, **dadurch gekennzeichnet, dass** die Nukleinsäure linear oder zirkulär ist.

## Revendications

1. Procédé d'hybridation d'au moins un oligonucléotide à un acide nucléique en fournissant l'acide nucléique avec au moins un oligonucléotide complémentaire, l'oligonucléotide fonctionnant comme un point de départ pour une polymérase pour la synthèse d'acide nucléique **caractérisé en ce que** l'oligonucléotide comprend au moins deux éléments photo-répondeurs qui changent d'une première à une seconde conformation après irradiation avec de la lumière, ne permettant pas ou permettant ainsi l'hybridation d'oligonucléotide.

2. Procédé selon la revendication 1 **caractérisé en ce que** l'acide nucléique sert de matrice pour la production d'un brin de nucléotide complémentaire par une polymérase créant ainsi un brin d'acide nucléique synthétisé.

3. Procédé selon la revendication 1 ou 2 **caractérisé en ce que** l'acide nucléique est circulaire et sert comme matrice pour l'amplification en cercle roulant.

4. Procédé selon l'une quelconque des revendications 1 à 3 **caractérisé en ce que**, l'oligonucléotide photo-répondeur fait partie d'un système de cadenas et l'acide nucléique sert de matrice pour la réaction de remplissage d'espace libre, l'espace libre du cadenas étant rempli avec des nucléotides complémentaires et ligué, une matrice circulaire étant formée.

5. Procédé selon la revendication 2 ou 4 **caractérisé en ce que** le brin d'acide nucléique synthétisé comprend l'oligonucléotide photo-répondeur, la conformation de l'oligonucléotide photo-répondeur étant changée à un état de non liaison après irradiation avec de la lumière, permettant ainsi à l'acide nucléique synthétisé de se dissocier de la matrice.

6. Procédé selon la revendication 1 **caractérisé en ce que** l'acide nucléique fourni sert de matrice pour une réaction de séquençage.

7. Procédé selon la revendication l'une quelconque des revendications 1 à 6 **caractérisé en ce que** la liaison de l'oligonucléotide est contrôlée par illumination spatiale de zones sans intérêt, changeant ainsi la conformation de l'oligonucléotide à un état de non liaison, contrôlant ainsi l'hybridation d'oligonucléotide à des sites spécifiques.

8. Procédé selon l'une quelconque des revendications 1 à 7 **caractérisé en ce que** la longueur de l'oligonucléotide photo-répondeur est d'au moins 15, 50 ou 75 nucléotides.

9. Procédé selon l'une quelconque des revendications 1 à 8 **caractérisé en ce que** la structure de l'oligonucléotide photo-répondeur a un rapport de X : N de 1 : 2 ou 1 : 3, N étant un nucléotide, X étant un élément photo-répondeur.

10. Procédé selon l'une quelconque des revendications 1 à 9 **caractérisé en ce que** l'oligonucléotide photo-répondeur a la formule N(XNN)nXN, N étant un nucléotide, X étant un élément photo-répondeur et n = 3 à 29.

11. Procédé selon l'une quelconque des revendications 1 à 10 **caractérisé en ce que** l'oligonucléotide photo-répondeur change d'une première à une seconde conformation après irradiation avec de la lumière ayant une première longueur d'onde et de la seconde à la première conformation après irradiation avec de la lumière ayant une seconde longueur d'onde.

12. Procédé selon la revendication 11 **caractérisé en ce que** la première longueur d'onde est comprise entre 300 et 400 nm et la seconde longueur d'onde est de 400 à 600 nm.

13. Procédé selon l'une quelconque des revendications 1 à 12 **caractérisé en ce que** l'acide nucléique est fourni par un échantillon de tissu, une puce ou un hydrogel.

14. Procédé selon l'une quelconque des revendications 1 à 13 **caractérisé en ce que** l'acide nucléique est linéaire ou circulaire.
